# EUROPEAN PATENT SPECIFICATION

(11) **EP 1 839 653 B1**
(45) Date of publication and mention of the grant of the patent: **09.06.2010**
(21) Application number: 06711866.1
(22) Date of filing: 18.01.2006
(51) Int. Cl.: A61K 31/122, A61P 25/02, A61P 25/04

(54) **THERAPEUTIC AGENT FOR SENSORY DISORDERS**
HEILMITTEL GEGEN SENSORISCHE ERKRANKUNGEN
AGENT THERAPEUTIQUE POUR LE TRAITEMENT D'UNE MALADIE SENSORIELLE

(30) Priority: 18.01.2005 JP 2005010858
(43) Date of publication of application: 03.10.2007
(60) Divisional application: 10003490.9
(73) Proprietor: Meiji Dairies Corporation, Koto-ku, Tokyo 136-8908 (JP)
(72) Inventor: SHIOMI, Hirohito, Fukuyama University, Hiroshima (JP); TAMURA, Yutaka, Fukuyama University, Fukuyama (JP); LUU, Bang, Centre Nat. de la Rech. Scientifique, 67084 Strasbourg (FR); YAMADA, M., Meiji Dairies Corp., Pharmaceut. Dept., Tokyo 136-8908 (JP); KOYAMA, K., Meiji Dairies Corp., Pharmaceut. Dept., Tokyo 136-8908 (JP); SUZUKI, H., Meiji Dairies Corp., Pharmaceut. Dept., Tokyo 136-8908 (JP)
(74) Representative: Grünecker, Kinkeldey, Stockmair & Schwanhäusser Anwaltssozietät
(86) International application number: PCT/JP2006/300597
(87) International publication number: WO 2006/077853

(56) References cited:
- WO-A-00/47199
- JP-A- 2000 297 034
- JP-A- 2001 089 404
- JP-A- 2001 213 771
- JP-A- 2001 515 058
- JP-A- 2002 068 973
- JP-A- 2002 241 270
- JP-A- 2002 241 271
- US-A1- 2003 203 040
- US-A1- 2004 102 527
- GONZALEZ DE AGUILAR JOSE-LUIS ET AL: "Neurotrophic activity of 2,4,4-trimethyl-3-(15- hydroxypentadecyl)-2-cyclohexen-1-one in cultured central nervous system neurons" BRAIN RESEARCH, ELSEVIER, AMSTERDAM, NL, vol. 920, no. 1-2, 1 January 2001 (2001-01-01), pages 65-73, XP009100736 ISSN: 0006-8993
- SAITO M ET AL: "Effect of cyclohexenonic long-chain fatty alcohol on rat overactive bladder induced by bladder neck obstruction" EUROPEAN JOURNAL OF PHARMACOLOGY, AMSTERDAM, NL, vol. 501, no. 1-3, 6 October 2004 (2004-10-06), pages 143-149, XP004587604 ISSN: 0014-2999
- LUU BANG ET AL: "Cyclohexenonic long-chain fatty alcohols as neuronal growth stimulators" MOLECULES, MOLECULAR DIVERSITY PRESERVATION INTERNATIONAL, BASEL, vol. 5, no. 12, 1 January 2000 (2000-01-01), pages 1439-1460, XP002181231 ISSN: 1420-3049
- APFEL S C ET AL: "Efficacy and safety of recombinant human nerve growth factor in patients with diabetic polyneuropathy: A randomized controlled trial" JOURNAL OF THE AMERICAN MEDICAL ASSOCIATION 20001101 US, vol. 284, no. 17, 1 November 2000 (2000-11-01), pages 2215-2221, XP002483660 ISSN: 0098-7484
- UNGER J W ET AL: "Nerve growth factor (NGF) and diabetic neuropathy in the rat: morphological investigations of the sural nerve, dorsal root ganglion, and spinal cord." EXPERIMENTAL NEUROLOGY SEP 1998, vol. 153, no. 1, September 1998 (1998-09), pages 23-34, XP002483661 ISSN: 0014-4886
- CHRISTIANSON J A ET AL: "Beneficial Actions of Neurotrophin Treatment on Diabetes-Induced Hypoalgesia in Mice" JOURNAL OF PAIN 200311 US, vol. 4, no. 9, November 2003 (2003-11), pages 493-504, XP002483662 ISSN: 1526-5900
- REN ET AL: "Nerve growth factor alleviates a painful peripheral neuropathy in rats" BRAIN RESEARCH, ELSEVIER, AMSTERDAM, NL, vol. 699, no. 2, 20 November 1995 (1995-11-20), pages 286-292, XP022256895 ISSN: 0006-8993
- TAMURA Y. ET AL.: 'Beneficial actions of MM-101 on diabetic hypoalgesia and neuropathic pain' J. PHARMACOL. SCI. vol. 97, no. SUPPLEMENT 1, 2005, page 171P, XP003004530
- GIRLANDA-JUNGES C. ET AL.: 'Effect of Cyclohexenonic Long Chain Fatty Alcohols on Neurite Outgrowth. Study on Structure-Activity Relationship' TETRAHEDRON vol. 54, 1998, pages 7735 - 7748, XP002198688
- SAH D.W.Y. ET AL.: 'NEUROTROPHIC FACTORS AS NOVEL THERAPEUTICS FOR NEUROPATHIC PAIN' NAT. REV. DRUG DISCOV. vol. 2, 2003, pages 460 - 472, XP003004531
- YAMAMOTO M. ET AL.: 'Shoko Byotai no Bunshi Mechanism Shinkei Suji Seishin Shoko Shojo Shibire Kankaku Shogai' MOL. MED. vol. 35, SPECIAL EXTRA ISSUE, December 1998, pages 520 - 522, XP003004532
- SUZUKI H.: 'Shinkei Insei Totsu to Shinkei Eiyo Inshi' BRAIN SCIENCE vol. 23, 2001, pages 837 - 844, XP003004533

## Description

The present invention relates to nonpeptide low molecular weight compounds which treat or prevent sensory disorders.

Pain is largely classified into two types, acute pain and chronic pain. Acute pain is a warning signal of particular types of damage or injury occurring in the body, such as an ankle sprain. By responding to the pain, this reaction attempts to remove the cause and prevent aggravation. On the other hand, chronic pain refers to continuous pain that is difficult to relieve by treatment. In particular, persisting pain of unknown apparent origin is referred to as "chronic intractable pain". Chronic pain, which is difficult to relieve, does not play a role as a warning signal to the living body since there is no action that can be taken to remove the cause.

Chronic intractable pain comprises of neurogenic pain, which arises from injury in or pressure on nerve tissue or the like, and nociceptive pain felt via the sensory receptors of sensory nerve endings. Mixed types of pain are also often found since increase in nociceptor sensitivity induces neurogenic pain. If pain occurs in the trigeminal nerve, it is called trigeminal neuralgia, and if pain occurs in the sciatic nerve, it is called sciatic neuralgia.

Furthermore, similar to pain, paralysis or torpor of sensitivity is also caused by nerve tissue injuries. For example, nerve tissue is injured either directly by physical damage, or indirectly by diseases. In other words, a hypersensitive state is pain and a hyposensitive state is paralysis or torpor. These symptoms are collectively referred to as sensory disorders.

Aside from traumas and after effects of surgery, sensory disorders can be caused by a variety of cancerous diseases, diabetic neuropathy, after effects of cerebral infarction, after effects of herpes zoster, phantom limb pain, spinal cord injuries, mononeuritis, mononeuropathy, polyneuropathy (such as heredopathia atactica polyneuritiformis, acute polyneuritis, Landry paralysis, Guillain-Barry syndrome, diphtheritic neuritis, toxic neuropathy, dystrophic neuropathy, collagenosis, and HIV), Hansen's disease, or the like.

For the treatment of pain, administration of anti-inflammatory analgesic drugs, nerve blockage, and treatment of causative diseases are performed. Nerve blockage and treatment of causative diseases are mostly performed for paralysis and/or torpor. These treatment techniques, however, are symptomatic and are far from being definitive cures. Moreover, regenerative medicine is being attempted in part, but it is still impractical.
[Non-Patent Document 1] Igaku no Ayumi (Journal of Clinical and Experimental Medicine) VOL. 211 P.606-609 NO. 5, 2004
[Non-Patent Document 2] Imura H, Ogata E, Takaku F, Tarui S (eds): Integrated Handbook of Internal Medicine VOL. 70 massyou/jiritsu shinkei shikkan (peripheral/autonomic nerve disorder), Nakayama-Shoten, pp. 211-215(1996)
[Non-Patent Document 3] Takashi Kanda "tahatsu shinkeien (polyneuritis)", [online], unknown date of publication, Yamaguchi University School of Medicine, Clinical Neuroscience (neurology), [searched on 12th January, 2005], internet <http://www.neurol.med.yamaguchi-u.ac.jp/sinryou/tahatsu.htm>
[Patent Document 1] Japanese Patent Application Kokai Publication No. (JP-A) 2000-297034 (unexamined, published Japanese patent application)
[Patent Document 2] JP-A 2002-241270
[Patent Document 3] JP-A 2002-241271

### Disclosure of the Invention

### Problems to be Solved by the Invention

An objective of the present invention is to provide low molecular weight compounds which treat sensory disorders.

### Means to Solve the Problems

It has been already reported that a cyclohexenone long chain alcohol has a neurotrophic function which promotes neuron survival and neurite extension (Gonzales de Aguilar et. al., Brain Res (2001) 920, 65-73). This time, the present inventors discovered that a cyclohexenone long chain alcohol represented by formula (1) improves sensory disorders, and completed the present invention.

The object is achieved by a compound for use in treating and/or preventing a sensory disorder, wherein said compound is a cyclohexenone long chain alcohol compound represented
by the following formula (1): 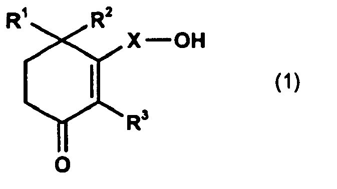 wherein
R¹, R², and R³ each represent a hydrogen atom or a methyl group, and
X represents a linear or branched C₁₀-C₂₈ alkylene or alkenylene group; and wherein the sensory disorder is reduction of pain threshold caused by nerve injury.

The object is also achieved by the use of a compound for the preparation of a medicament for treating and/or preventing a sensory disorder, wherein said compound is a cyclohexenone long chain alcohol compound represented by the following formula (1): 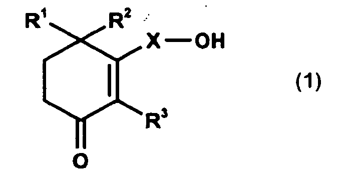 wherein
R¹, R², and R³ each represent a hydrogen atom or a methyl group, and
X represents a linear or branched C₁₀-C₂₈ alkylene or alkenylene group; and wherein the sensory disorder is reduction of pain threshold caused by nerve injury.

Preferrred embodiments are set forth in the subclaims.

The compound of formula (1) is preferably a cyclohexenone long chain alcohol compound selected from the group consisting of the following compounds:
3-(10-hydroxydecyl)-2-cyclohexen-1-one (3-(10-hydroxydecyl)-2-cyclohexenone);
3-(11-hydroxyundecyl)-2-cyclohexen-1-one (3-(11-hydroxyundecyl)-2-cyclohexenone);
3-(12-hydroxydodecyl)-2-cyclohexen-1-one (3-(12-hydroxydodecyl)-2-cyclohexenone);
3-(13-hydroxytridecyl)-2-cyclohexen-1-one (3-(13-hydroxytridecyl)-2-cyclohexenone);
3-(14-hydroxytetradecyl)-2-cyclohexen-1-one (3-(14-hydroxytetradecyl)-2-cyclohexenone);
3-(10-hydroxydecyl)-4-methyl-2-cyclohexen-1-one (3-(10-hydroxydecyl)-4-methyl-2-cyclohexenone);
3-(11-hydroxyundecyl)-4-methyl-2-cyclohexen-1-one (3-(11-hydroxyundecyl)-4-methyl-2-cyclohexenone);
3-(12-hydroxydodecyl)-4-methyl-2-cyclohexen-1-one (3-(12-hydroxydodecyl)-4-methyl-2-cyclohexenone);
3-(13-hydroxytridecyl)-4-methyl-2-cyclohexen-1-one (3-(13-hydroxytridecyl)-4-methyl-2-cyclohexenone);
3-(14-hydroxytetradecyl)-4-methyl-2-cyclohexen-1-one (3-(14-hydroxytetradecyl)-4-methyl-2-cyclohexenone);
4,4-dimethyl-3-(10-hydroxydecyl)-2-cyclohexen-1-one (4,4-dimethyl-3-(10-hydroxydecyl)-2-cyclohexenone);
3-(11-hydroxyundecyl)-4,4-dimethyl-2-cyclohexen-1-one (3-(11-hydroxyundecyl)-4,4-dimethyl-2-cyclohexenone);
3-(12-hydroxydodecyl)-4,4-dimethyl-2-cyclohexen-1-one (3-(12-hydroxydodecyl)-4,4-dimethyl-2-cyclohexenone);
3-(13-hydroxytridecyl)-4,4-dimethyl-2-cyclohexen-1-one (3-(13-hydroxytridecyl)-4,4-dimethyl-2-cyclohexenone);
3-(14-hydroxytetradecyl)-4,4-dimethyl-2-cyclohexen-1-one (3-(14-hydroxytetradecyl)-4,4-dimethyl-2-cyclohexenone);
3-(10-hydroxydecyl)-2-methyl-2-cyclohexen-1-one (3-(10-hydroxydecyl)-2-methyl-2-cyclohexenone);
3-(11-hydroxyundecyl)-2-methyl-2-cyclohexen-1-one (3-(11-hydroxyundecyl)-2-methyl-2-cyclohexenone);
3-(12-hydroxydodecyl)-2-methyl-2-cyclohexen-1-one (3-(12-hydroxydodecyl)-2-methyl-2-cyclohexenone);
3-(13-hydroxytridecyl)-2-methyl-2-cyelohexen-1-one (3-(13-hydroxytridecyl)-2-methyl-2-cyclohexenone);
3-(14-hydroxytetradecyl)-2-methyl-2-cyclohexen-1-one (3-(14-hydroxytetradecyl)-2-methyl-2-cyclohexenone);
3-(12-hydroxydodecyl)-2,4,4-trimethyl-2-cyclohexen-1-one (3-(12-hydroxydodecyl)-2,4,4-trimethyl-2-cyclohexenone);
3-(13-hydroxytridecyl)-2,4,4-trimethyl-2-cyclohexen-1-one (3-(13-hydroxytridecyl)-2,4,4-trimethyl-2-cyclohexenone);
3-(14-hydroxytetradecyl)-2,4,4-trimethyl-2-cyclohexen-1-one (3-(14-hydroxytetradecyl)-2,4,4-trimethyl-2-cyclohexenone);
3-(15-hydroxypentadecyl)-2,4,4-trimethyl-2-cyclohexen-1-one (3-(15-hydroxypentadecyl)-2,4,4-trimethyl-2-cyclohexenone); and
3-(16-hydroxyhexadecyl)-2,4,4-trimethyl-2-cyclohexen-1-one (3-(16-hydroxyhexadecyl)-2,4,4-trimethyl-2-cyclohexenone).

Furthermore, the present invention provides medical packages comprising the following components i and ii:
i. a medical composition comprising the cyclohexenone long chain alcohol compound represented by formula (1) and a pharmaceutically acceptable carrier, and
ii. instructions which describe that the above medical composition can be used in either the prevention or the treatment of a sensory disorder wherein the sensory disorder is reduction of pain threshold caused by nerve injury, or both.

The instructions of the present invention may describe the date of manufacture of the medical composition and storage conditions, as well as the subject of treatment. Furthermore, such information may also be displayed directly on the medical composition. Specifically, necessary information can be displayed by directly printing on a container filled with the medical composition, or by pasting a label thereon. That is, the present invention relates to medical compositions comprising the cyclohexenone long chain alcohol compound represented by formula (1) and a pharmaceutically acceptable carrier, with instructions showing that the medical composition can be used in either the prevention or treatment of a sensory disorder wherein the sensory disorder is reduction of pain threshold caused by nerve injury, or both.

### Effects of the Invention

The compound represented by formula (1) was confirmed to have a relieving or preventive effect on the symptoms of sensory disorders in model animals having sensory disorders. Accordingly, the compound represented by formula (1) is useful for preventing and treating a sensory disorder wherein the sensory disorder is reduction of pain threshold caused by nerve injury. The present invention can significantly improve the quality of life (QOL) of patients with sensory disorders.

### Brief Description of the Drawings

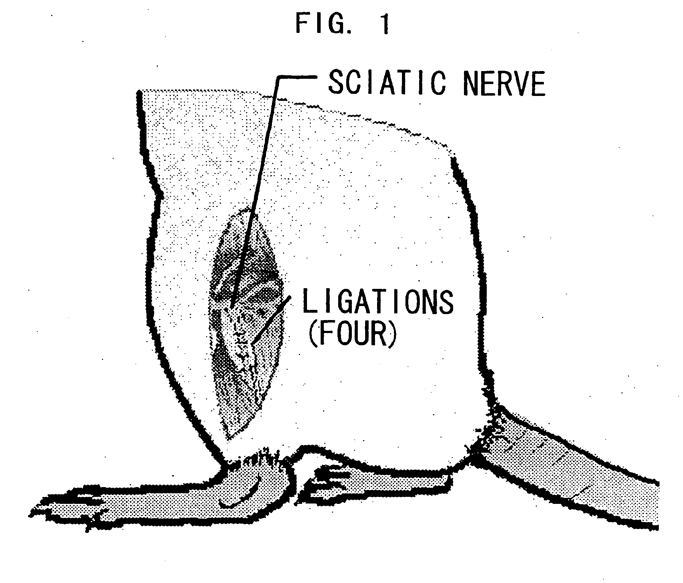

Fig. 1 depicts a method for making a Bennett model. The position of the sciatic nerve axon to be ligated and the ligation sites thereof are shown.

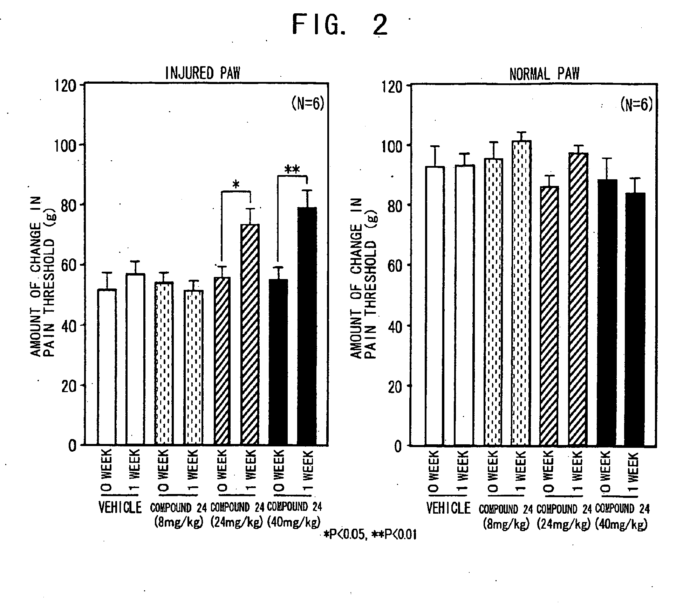

Fig. 2 depicts the therapeutic effect of compound 24 on the reduction of pain threshold in Bennett model (hyperalgesia model) rats. The left graph (injured paw) shows the mean ± standard error of the amount of change in pain threshold of the nerve-injured paw (operated side). The right graph (normal paw) shows the mean ± standard error of the amount of change in pain threshold of the normal paw (untreated side). *:p<0.05, **:p<0.01 (Student's t-test)

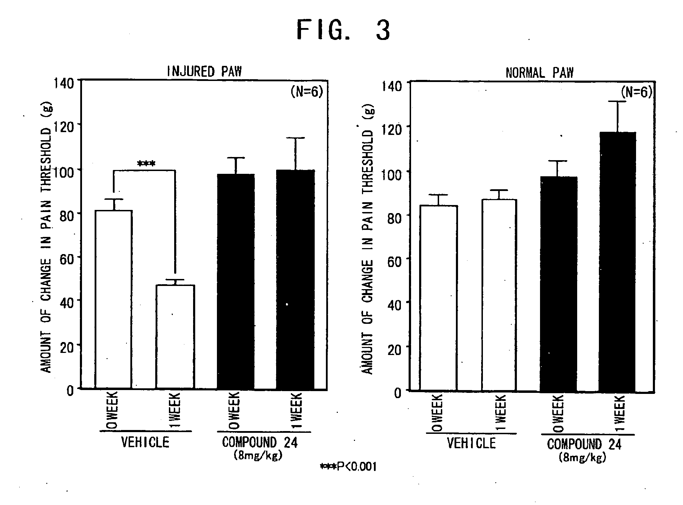

Fig. 3 depicts the preventive effect of compound 24 on the reduction of pain threshold in Bennett model (hyperalgesia model) rats. The left graph (injured paw) shows the mean ± standard error of the amount of change in pain threshold of the nerve-injured paw (operated side). The right graph (normal paw) shows the mean ± standard error of the amount of change in pain threshold of the normal paw (untreated side). ***:p<0.001 (Student's t-test)

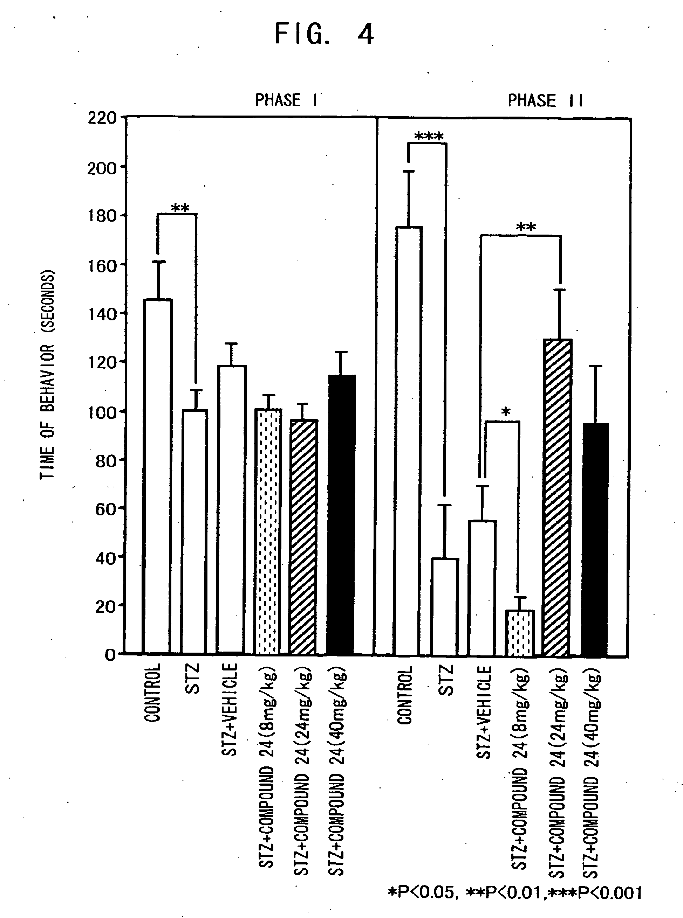

Fig. 4 depicts the improving effect of compound 24 on dullness in STZ-induced diabetic model mice. The mean ± standard error of the total time spent for licking behavior and biting behavior in Phase I and Phase II, is shown. *:p<0.05, **:p<0.01, ***:p<0.001 (Student's t-test).

### Best Mode for Carrying Out the Invention

In the above formula (1), X represents a linear or branched C10-C28 alkylene or alkenylene group, and examples of the side chains of the branched alkylene or alkenylene group include C1-C10 alkyl groups. Examples of the side chain alkyl groups include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, an isohexyl group, a heptyl group, an octyl group, a nonyl group, and a decyl group. Of these, a methyl group is particularly preferred.

The linear alkylene group or alkenylene group, which refers to an alkene structure having at least one carbon-carbon double bond, is preferably substituted at positions 3 and/or 7 of the side chain. In other words, if X in formula (1) is a linear alkylene group or a linear alkenylene group, side chains may be included at either one, or both, of positions 3 and 7. Of these Xs, a linear C10-C28 alkylene group is more preferred, and a linear C10-C18 alkylene group is particularly preferred. Moreover, R¹, R², and R³ each represent a hydrogen atom or a methyl group, while it is more preferred that at least any one of the R¹, R², and R³ is a methyl group. Furthermore, compounds in which R¹, R², and R³ are all methyl groups are more preferred compounds in the present invention. In another embodiment, cases in which R¹ and R² are both hydrogen atoms are also preferred.

Moreover, the compound of formula (1) may be in the form of a pharmaceutically acceptable salt, or a solvate or hydrate thereof. The compound of formula (1) has a variety of possible isomers, which are also encompassed by the present invention.

The method for obtaining the compound represented by formula (1) is publicly known. For example, the compound can be produced according to the process described in JP-A 2000-297034. More specifically, the compound represented by formula (1) can be produced according to, for example, the following process A or B.

### [Process A]

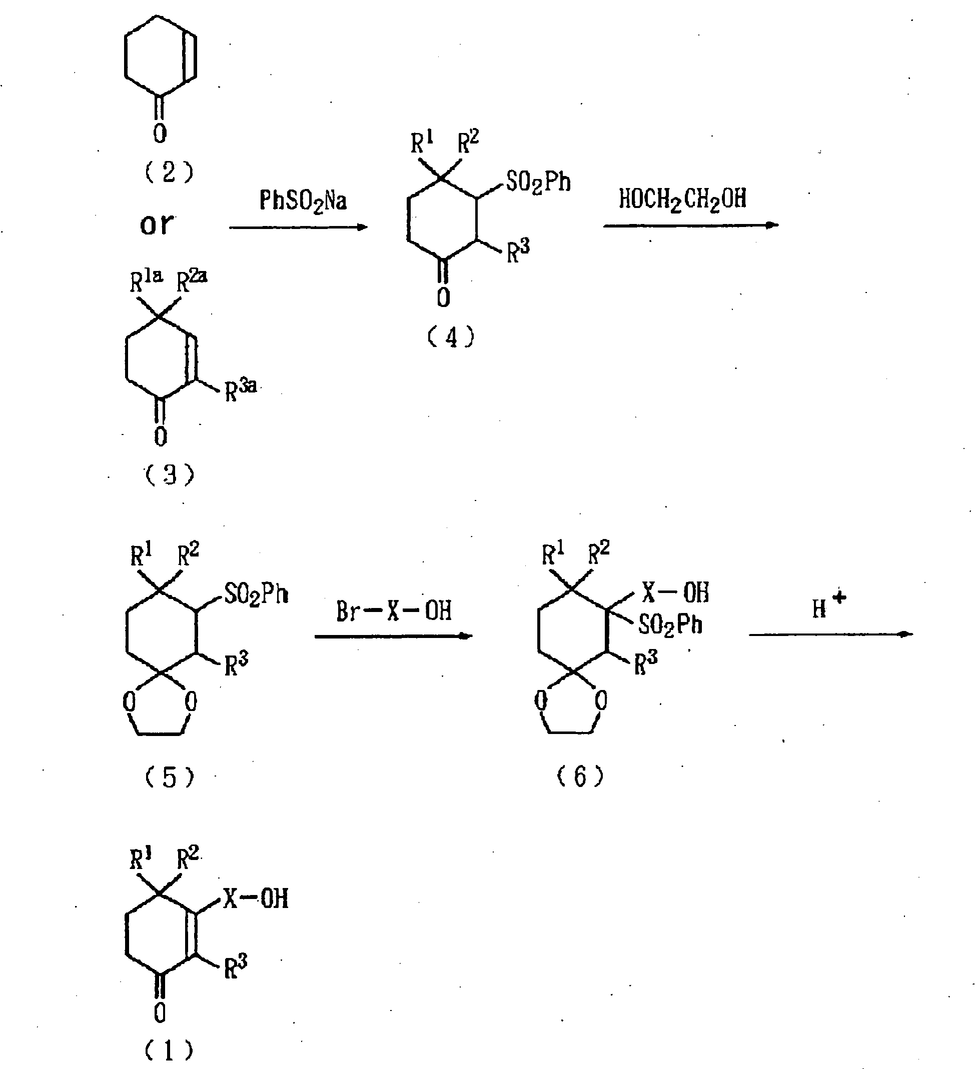

[wherein R^{1a}, R^{2a}, and R^{3a} represent a hydrogen atom or a methyl group. At least one of R^{1a}, R^{2a}, and R^{3a} represents a methyl group. Ph represents a phenyl group, and X, R¹, R², and R³ have the same meanings as defined above.]

That is, cyclohexenone (2) or methyl-substituted-2-cyclohexen-1-one (3) is reacted with a benzenesulfinic acid salt in the presence of an acid, to yield compound (4). The resulting compound (4) is reacted with ethylene glycol to obtain its ketal derivative (5), which is further reacted with ω-halogenoalkanol or ω-halogenoalkenol to yield compound (6). The obtained compound (6) is subjected to an acid treatment to eliminate the protective group, and thereby compound (1) is obtained.

The methyl-substituted-2-cyclohexen-1-one (3) used here as a raw material can be obtained by reacting methyl-substituted cyclohexanone with a trialkylsilyl halide in the presence of butyl lithium, followed by oxidation in the presence of a palladium-based catalyst.

First, the reaction between cyclohexenone (2) or methyl-substituted-2-cyclohexen-1-one (3) and a benzenesulfinic acid salt, for example, benzenesulfinic acid sodium is preferably performed in the presence of an acid such as hydrochloric acid, sulfuric acid, and phosphoric acid at 0 to 100°C for 5 to 40 hours.

The reaction between compound (4) and ethylene glycol is preferably performed in the presence of a condensing agent such as paratoluenesulfonic anhydride at 50 to 120°C for 1 to 10 hours.

The ω-halogenoalkanol or ω-halogenoalkenol to be reacted with the ketal derivative (5) is preferably ω-bromoalkanol or ω-bromoalkenol. The reaction between the ketal derivative (5) and ω-halogenoalkanol or ω-halogenoalkenol is preferably performed in the presence of a metal compound such as butyl lithium in low-temperature conditions.

The phenylsulfonyl group and the ketal-protective group of the obtained compound (6) can be eliminated by reacting compound (6) with an acid such as paratoluenesulfonic acid.

### [Process B]

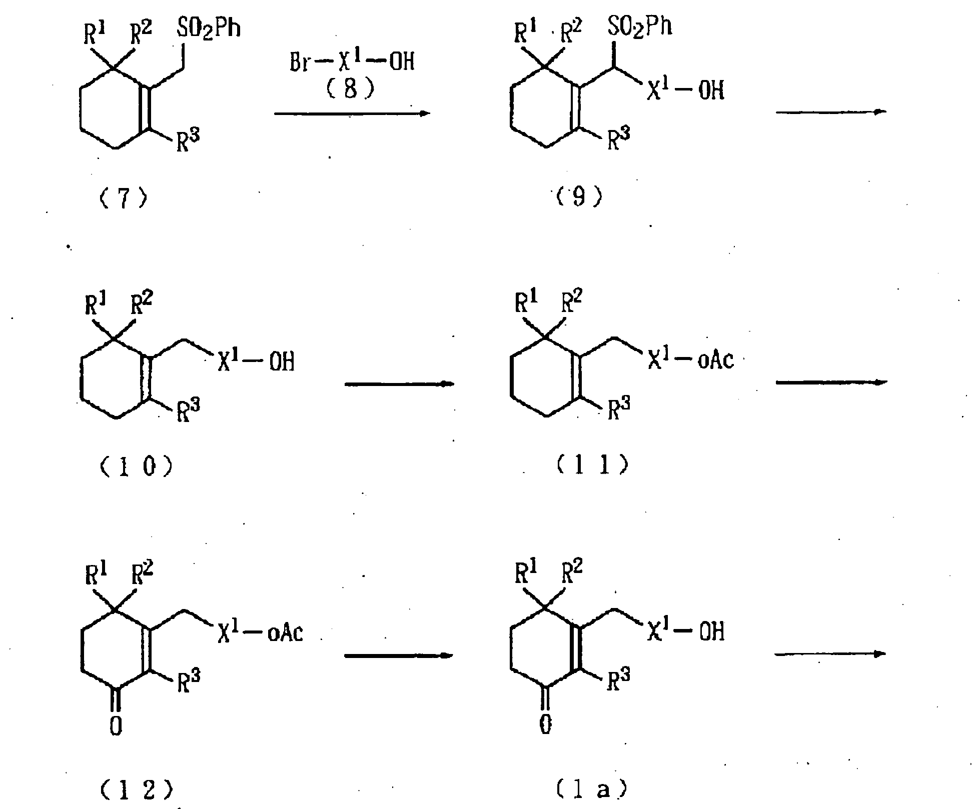

[wherein X¹ represents C9-C27 alkylene group or alkenylene group, Ac represents an acyl group, and R¹, R², R³, and Ph have the same meanings as defined above.]

That is, compound (7) is reacted with ω-bromoalcohol to yield compound (9), followed by elimination of the phenylsulfonyl group to obtain compound (10). Compound (7) can be obtained in accordance with, for example, Synthesis, 1996, Nov. The hydroxy group of the obtained compound (10) is protected to yield compound (11), followed by oxidation to yield compound (12). Furthermore, the hydroxy-protective group of compound (12) is eliminated to thereby obtain compound (1a).

The reaction between compound (7) and compound (8) is preferably performed in the presence of a metal compound such as butyl lithium under low-temperature conditions. The phenylsulfonyl group can be eliminated from compound (9) preferably by reacting compound (9) with, for example, a phosphate salt in the presence of sodium amalgam. The hydroxy-protective group of compound (10) is preferably an acetyl group or the like, and the protection reaction is performed, for example, by reacting compound (10) with acetic anhydride. The oxidation reaction of compound (11) is performed by reacting compound (11) with an alkyl hydroperoxide such as t-butyl hydroperoxide in the presence of a metal compound such as ruthenium trichloride. The protective group can be eliminated from compound (12) preferably by hydrolyzing compound (12) in the presence of a base such as potassium carbonate.

In the present invention, the terms "treatment" and "therapy" include prevention. In patients with chronic diseases such as diabetes, the pathological condition is continuous. Therefore, a drug administered for the purpose of treatment gives a therapeutic effect on the pathological condition that has been continuing from prior to administration. At the same time, the drug preventively acts on pathological condition(s) that occur administration.

The abnormal enhancement of sensitivity causes hypersensitivity. Patients with hypersensitivity experience strong pain. On the other hand, the abnormal suppression of sensitivity causes paralysis or torpor. The term "paralysis" refers to the condition where the sense of perception is lost. The sense includes visual sense, thermal sense, cutaneous sense of touch, auditory sense, olfactory sense, sense of taste, sense of pain, and such. The term "paralysis" refers to the complete suppression of perception, whereas the term "torpor" includes a degree of suppression that is incomplete. "Torpor" includes cases where a certain degree of perception remains. "Paralysis" refers to a state of torpor in which the degree of sensitivity suppression is high. In other words, "torpor" includes "paralysis".

Moreover, the term "reduction of pain threshold caused by nerve injuries" refers to a state in which nerve injuries lower the threshold to mechanical stimuli or thermal stimuli, and thus the perception/sense of pain is supersensitized (Imura H, Ogata E, Takaku F, Tarui S (eds): "Integrated Handbook of Internal Medicine VOL. 70 massyou/jiritsu shinkei shikkan (peripheral/autonomic nerve disorder)", Nakayama-Shoten, pp. 211-215(1996)). In the present invention, the term "reduction of pain threshold" includes "reduction of pain tolerance level".

The term "polyneuropathy" refers to a dysfunction which simultaneously and systemically occurs in a lot of peripheral nerves. The sensitivity is destroyed, causing symptoms such as stinging pain, numbness, burning pain, loss of vibratory sensations, and loss of positional sense of limbs or the like (including walking difficulty and difficulty in maintenance of posture). Moreover, disorder in the autonomic nervous system causes symptoms such as constipation, uncontrollability of intestines or bladder (including incontinence of urine and feces), sexual dysfunction, unusual fluctuation of blood pressure, and orthostatic hypotension.

It is known that acute "polyneuropathy" is brought by, for example, the following factors:
infectious diseases caused by endotoxin- or exotoxin-producing bacteria or the like (such as diphtheria);
autoimmune responses (such as Guillain-Barry syndrome);
toxic materials (such as heavy metals);
carbon monoxide;
phenytoin;
antibiotics (such as chloramphenicol, nitrofurantoin, and sulfonamide);
chemotherapeutic drugs (such as vinblastin and vincristin);
sedative drugs (such as barbital and hexobarbital); and
cancers (such as multiple myeloma).

Alternatively, the causative factors of chronic polyneuropathy include those described below; however, chronic polyneuropathy is often caused by unknown origins.
diabetes;
excessive intake of alcohol;
nutritional deficiencies (deficiency of vitamin B and the like);
pernicious anemia (due to deficiency of vitamin B 12);
dysthyreosis;
hepatic failure;
renal failure;
cancer; and
excessive intake of vitamin B6 (pyridoxine).

In particular, distal polyneuropathy occurs in diabetic polyneuropathy. Pain is aggravated by mechanical stimuli and temperature change. Moreover, since the thermal sense and the sense of pain are lost, external stimuli becomes difficult to perceive, thus causing burns, ulcers due to trauma or pressure on skin, Charcot's joints, and the like. Furthermore, some polyneuropathies are hereditary (Fukushima Masanori (editorial supervisor), The Merck Manual of Medical Information-Home Edition, Japan Banyu Pharmaceutical Co, Ltd., pp. 343-345(1999)).

In addition, it is known that various neuropathies are found in traumas, sequelae of surgery, a variety of cancerous diseases, diabetic neuropathy, sequelae of cerebral infarction, sequelae of herpes zoster, phantom limb pain, spinal cord injuries, mononeuritis, mononeuropathy, polyneuropathy, and Hansen's disease. Polyneuropathy includes neurological symptoms brought by hereditary neuritis, acute polyneuritis, Landry paralysis, Guillain-Barry syndrome, diphtheritic neuritis, toxic neuropathy, dystrophic neuropathy, collagenosis, HIV infection, or the like.

The term "mononeuropathy" refers to a nerve disorder in a specific site, whereas the abovementioned polyneuropathy is a systemic symptom. Typically, it is brought by a nerve disorder involved in trauma or tissue damage. Mononeuropathy includes, for example, a nerve disorder caused by pressure. More specifically, compression neuropathy is a representative mononeuropathy. The Bennett model described later is an appropriate model of compression neuropathy.

The present inventors confirmed by the following experiments that the compound having formula (1) has a preventive effect and a therapeutic effect on various types of neuropathies.
Example 1: analgesic action in nerve injury models
Example 2: injury-preventive action in nerve injury models
Example 3: improving action in dullness models

For example, in a Bennett model (sciatic nerve injury model), thermal hyperalgesia and mechanical allodynia (paralgesia) occur, and particularly, a strong onset of thermal hyperalgesia is seen. In Example 1, Bennett models are made, and after confirming hyperalgesia, administration of compound 24 is started, and evaluation is performed using as an index the pain threshold to a pressure stimulus. This supports the therapeutic effect on mechanical allodynia (paralgesia) (Bennett GJ et. al., Pain, 33(1), pp. 87-107(1988)).

Moreover, in Example 2, Bennett models are made, and prior to hyperalgesia onset, administration of a low dosage of compound 24 is started, and evaluation is performed using as an index the pain threshold to a pressure stimulus. Thus, this result supports the preventive effect of the compound on mechanical allodynia (paralgesia).

Furthermore, in Example 3, production of diabetes models and administration of compound 24 are started at the same time, and pain caused by a constant chemical stimulus is evaluated. This result supports the preventive effect on polyneuropathy.

The present compound has been found to have the effect of extending neurites or inducing the differentiation of nerve stem cells into neurons; thus it is expected to directly act on nerves, providing a definitive cure for sensory disorders.

In the present invention, compound (1) may be administered by an arbitrary method, when it is used to prevent and/or treat these neuropathies. Specifically, compound (1) may be administered by oral administration or parenteral administration. Parenteral administration includes administration forms such as intramuscular administration, subcutaneous administration, intravenous administration, or administration by use of a suppository.

If a formulation for oral administration is to be prepared, excipients and other additives such as binders, disintegrators, lubricants, colorants, and flavoring agents are added as required, and then formed into tablets, coated-tablets, granules, capsules, solutions, syrups, elixirs, oily or aqueous suspensions, or the like by ordinary methods. Examples of excipients include lactose, com starch, saccharose, glucose, sorbitol, and crystalline cellulose. Examples of binders include polyvinyl alcohol, polyvinyl ether, ethyl cellulose, methyl cellulose, gum arabic, tragacanth, gelatin, shellac, hydroxypropyl cellulose, hydroxypropyl starch, and polyvinyl pyrrolidone.

Examples of disintegrators include starch, agar, gelatin powder, crystalline cellulose, calcium carbonate, sodium bicarbonate, calcium citrate, dextran, and pectin. Examples of lubricants include magnesium stearate, talc, polyethylene glycol, silica, and hardened vegetable oil. Colorants that are permitted to be added to pharmaceuticals may be used. Examples of flavoring agents which may be used include cocoa powder, menthol crystal, aromatic acid, menthol oil, borneol, cinnamon powder, menthol, peppermint oil, and camphor. These tablets and granules may be appropriately coated with sugar, gelatin, and other coatings as required.

If an injection is prepared, pH-regulators, buffers, stabilizers, preservatives, and the like are added as required, and formed into a formulation for subcutaneous, intramuscular, or intravenous injection, by ordinary methods. The injection may be in a solid form, by for example, lyophilizing the solution after it is stored into a container, to be prepared as needed. Moreover, a single dose may be stored in a container, or a plurality of doses may be stored in the same container.

When the compound of the present invention is administered as a pharmaceutical, the daily dose for a human adult is typically within the range of 0.01 to 1000 mg, and preferably the range of 0.1 to 500 mg. The daily dose is administered either at a single time or in 2-4 divided doses.

### [EXAMPLES]

### Preparation Example 1:

(1) 10.25 g of benzenesulfinic acid sodium was added to a solution having 5 ml of cyclohexenone and 30 ml of water. To this solution, 60 ml of 1N hydrochloric acid was added dropwise. After stirring at room temperature for 24 hours, the crystals thus deposited were filtered, and washed with water, isopropanol, and cold ether. After recrystallization with isopropanol, 5.74 g of 3-(phenylsulfonyl)-cyclohexan-1-one was obtained in the form of white crystals (Melting Point: 83 to 85°C) (Yield: 97%).
(2) 0.3 ml of 1,2-ethanediol and 0.2 g of anhydrous paratoluenesulfonic acid were added to a solution having 5.3 g of 3-(phenylsulfonyl)-cyclohexan-1-one dissolved in 60 ml of benzene. The reaction solution was heated under reflux for four hours. After the reaction, 2M aqueous sodium bicarbonate solution was added thereto, and extraction done three times with ethyl acetate. The organic phase was washed with saturated saline, and then dried over magnesium sulfate. The solvent was distilled off under reduced pressure. Then, the residue was recrystallized with ether, to thereby obtain 6.1 g of 1,1-(ethylenedioxy)-3-(phenylsulfonyl)-cyclohexane in the form of white crystals (Melting Point: 93 to 95°C) (Yield: 97%).
(3) 2 ml of n-butyl lithium solution was added dropwise to 5 ml of THF (tetrahydro furan) solution having 565 mg of 1,1-(ethylenedioxy)-3-(phenylsulfonyl)-cyclohexane and 4 mg of triphenylmethane at -78°C under an argon stream. After stirring for 10 minutes, the reaction was effected at room temperature for one hour. 1 ml of HMPT (hexamethyl phosphoric triamide) was added thereto. The resulting solution was recooled to -78°C, and 2 ml of THF solution having 159 mg of 10-bromo-1-decanol was added dropwise. After the reaction at - 20°C for two hours, the reaction solution was poured into a saturated ammonium chloride solution. The solution was extracted with ether. The organic phase was washed with water and saturated saline, and then dried over magnesium sulfate. The solvent was distilled off under reduced pressure. Then, the residue was purified by silica gel column chromatography using hexane-ethyl acetate (AcOEt), to thereby obtain 265 mg of 1,1-(ethylenedioxy)-3-(10-hydroxydecyl)-3-(phenylsulfonyl)-cyclohexane in the form of a colorless oil (Yield: 90%).
(4) 20 mg of paratoluenesulfonic acid was added to a solution having 193 mg of 1,1-(ethylenedioxy)-3-(10-hydroxydecyl)-3-(phenylsulfonyl)-cyclohexane in 3 ml of chloroform and 0.6 ml of acetone. The mixed solution was reacted at 50°C for 24 hours. 10 ml of saturated aqueous sodium bicarbonate solution was added thereto, and subjected to dichloromethane extraction. The organic phase was washed with saturated saline, and then dried over magnesium sulfate. The solvent was distilled off under reduced pressure. Then, the residue was purified by silica gel column chromatography using hexane-ethyl acetate, to thereby obtain 86 mg of 3-(10-hydroxydecyl)-2-cyclohexen-1-one (3-(10- hydroxydecyl)-2-cyclohexenone) in the form of a colorless oil (Yield: 77%).

In a similar manner to Preparation Example 1, the following compounds were obtained.

### Preparation Example 2:

3-(11-hydroxyundecyl)-2-cyclohexen-1-one (3-(11-hydroxyundecyl)-2-cyclohexenone) (Melting Point: 34 to 35°C).

### Preparation Example 3:

3-(12-hydroxydodecyl)-2-cyclohexen-1-one (3-(12-hydroxydodecyl)-2-cyclohexenone) (Melting Point: 35 to 36°C)

### Preparation Example 4:

3-(13-hydroxytridecyl)-2-cyclohexen-1-one (3-(13-hydroxytridecyl)-2-cyclohexenone) (Melting Point: 42 to 43°C)

### Preparation Example 5:

3-(14-hydroxytetradecyl)-2-cyclohexen-1-one (3-(14-hydroxytetradecyl)-2-cyclohexenone) (Melting Point: 44 to 45°C)

### Preparation Example 6:

(1) 35.4 ml of 1.4M n-butyl lithium solution was added dropwise to 20 ml of THF solution having 7 ml of N,N-diisopropylamine at -78°C. The solution was stirred at 0°C for 30 minutes. This LDA (lithium diisopropylamide) solution was added dropwise to 10 ml of THF solution having 4 ml of 4-methylcyclohexan-1-one at -78°C. After stirring at -78°C for one hour, 6.5 ml of trimethylsilyl chloride was added thereto. After stirring at room temperature for one hour, the solution was poured into an aqueous sodium bicarbonate solution, and extracted with ether. The organic phase was washed with saturated saline, and then dried over magnesium sulfate. The solvent was distilled off under reduced pressure. Then, the residue was purified by vacuum distillation, to thereby obtain 5.83 g of 4-methyl-1-(trimethylsilyloxy)-1-cyclohexene (thin layer chromatography/TLC:(hexane-AcOEt:8-2)Rf=0.8) (Yield: 96%).
(2) A catalyst amount of palladium acetate was added to 70 ml of DMSO (dimethylsulfoxide) solution having 3.53 g of 4-methyl-1-(trimethylsilyloxy)-1-cyclohexene, followed by stirring while introducing oxygen for six hours. Water was added at 0°C, and the solution was filtered over celite, and then extracted with ether. The solvent of the organic phase was distilled off under reduced pressure and the residue was dissolved in hexane-water, and the resulting solution was extracted with hexane. The hexane phase was washed with saturated saline and dried over magnesium sulfate. The solvent was distilled off under reduced pressure, to thereby obtain 4-methyl-2-cyclohexen-1-one in the form of an oil (TLC:(hexane-AcOEt:8-2)Rf=0.35) (Yield: 72%).
(3) 3.0 g of benzenesulfinic acid sodium was added to a solution containing 1.52 g of 4-methyl-2-cyclohexen-1-one and 9 ml of water. 18 ml of 1N hydrochloric acid was added dropwise to this solution. After stirring at room temperature for 24 hours, the crystals thus deposited were filtered, and washed with water, isopropanol, and cold ether. After recrystallization with isopropanol, 4-methyl-3-(phenylsulfonyl)-cyclohexan-1-one (Melting Point: 71 to 74°C) was obtained in the form of white crystals (Yield: 72%).
(4) 0.7 ml of 1,2-ethanediol and 0.2 g of anhydrous paratoluenesulfonic acid were added to a solution having 2.45 g of 4-methyl-3-(phenylsulfonyl)-cyclohexan-1-one in 40 ml of benzene. The reaction solution was heated under reflux for four hours. After the reaction, 2M aqueous sodium bicarbonate solution was added thereto, and extraction done three times with ethyl acetate. The organic phase was washed with saturated saline, and then dried over magnesium sulfate. The solvent was distilled off under reduced pressure. Then, the residue was recrystallized with ether, to thereby obtain 1,1-(ethylenedioxy)-4-methyl-3-(phenylsulfonyl)-cyclohexane (Melting Point: 105 to 106°C) in the form of white crystals (Yield: 97%).
(5) 1.8 ml solution of n-butyl lithium was added dropwise to 5 ml of THF solution having 560 mg of 1,1-(ethylenedioxy)-4-methyl-3-(phenylsulfonyl)-cyclohexane and 4 mg of triphenylmethane at -78°C under an argon stream. After stirring for 10 minutes, the reaction was effected at room temperature for one hour. 1 ml of HMPT was added thereto. The resulting solution was recooled to -78°C, and 2 ml of THF solution having 166 mg of 10-bromo-1-decanol was added dropwise. After reaction at -20°C for two hours, the reaction solution was poured into a saturated ammonium chloride solution. The solution was extracted with ether. The organic phase was washed with water and saturated saline, and then dried over magnesium sulfate. The solvent was distilled off under reduced pressure. The residue was then purified by silica gel column chromatography using hexane-ethyl acetate, to thereby obtain 1,1-(ethylenedioxy)-3-(10-hydroxydecyl)-4-methyl-3-(phenylsulfonyl)-cyclohexane in the form of a colorless oil (TLC:(hexane-AcOEt:6-4)Rf=0.14) (Yield: 97%).
(6) 20 mg of paratoluenesulfonic acid was added to a solution having 235 mg of 1,1-(ethylenedioxy)-3-(10-hydroxydecyl)-4-methyl-3-(phenylsulfonyl)-cyclohexane in 20 ml of chloroform and 4 ml of acetone. The mixed solution was reacted at 50°C for 24 hours. 10 ml of saturated aqueous sodium bicarbonate solution was added thereto, and extraction done with dichloromethane. The organic phase was washed with saturated saline, and then dried over magnesium sulfate. The solvent was distilled off under reduced pressure. Then, the residue was purified by silica gel column chromatography using hexane-ethyl acetate, to thereby obtain 3 -(10-hydroxydecyl)-4-methyl-2-cyclohexen-1-one (3-(10-hydroxydecyl)-4-methyl-2-cyclohexenone) in the form of a colorless oil (TLC:(hexane-AcOEt:6-4)Rf=0.2) (Yield: 75%).

In a similar manner to Preparation Example 6, the following compounds were obtained.

### Preparation Example 7:

3-(11-hydroxyundecyl)-4-methyl-2-cyclohexen-1-one (3-(11-hydroxyundecyl)-4-methyl-2-cyclohexenone) (TLC:(hexane-AcOEt:6-4)Rf=0.21)

### Preparation Example 8:

3-(12-hydroxydodecyl)-4-methyl-2-cyclohexen-1-one (3-(12-hydroxydodecyl)-4-methyl-2-cyclohexenone) (TLC:(hexane-AcOEt:6-4)Rf=0.22)

### Preparation Example 9:

3-(13-hydroxytridecyl)-4-methyl-2-cyclohexen-1-one (3-(13-hydroxytridecyl)-4-methyl-2-cyclohexenone) (TLC:(hexane-AcOEt:6-4)Rf=0.25)

### Preparation Example 10:

3-(14-hydroxytetradecyl)-4-methyl-2-cyclohexen-1-one (3-(14-hydroxytetradecyl)-4-methyl-2-cyclohexenone) (TLC:(hexane-AcOEt:6-4)Rf=0.3)

### Preparation Example 11:

(1) 5.98 g of benzenesulfinic acid sodium was added to a solution having 3 ml of 4,4-dimethyl-2-cyclohexen-1-one and 30 ml of water. 40 ml of 1N hydrochloric acid was added dropwise to this solution. After stirring at room temperature for 24 hours, the crystals thus deposited were filtered, and washed with water, isopropanol, and cold ether. After recrystallization with isopropanol, 4,4-dimethyl-3-(phenylsulfonyl)-cyclohexan-1-one was obtained in the form of white crystals (Melting Point: 84 to 86°C) (Yield: 89%).
(2) 1.1 ml of 1,2-ethanediol and 0.3 g of anhydrous paratoluenesulfonic acid were added to a solution having 4.4 g of 4,4-dimethyl-3-(phenylsulfonyl)-cyclohexan-1-one dissolved in 45 ml of benzene. The reaction solution was heated under reflux for four hours. After the reaction, 2M aqueous sodium bicarbonate solution was added thereto, and extracted with ethyl acetate three times. The organic phase was washed with saturated saline, and then dried over magnesium sulfate. The solvent was distilled off under reduced pressure. Then, the residue was recrystallized with ether, to thereby obtain 4,4-dimethyl-1,1-(ethylenedioxy)-3-(phenylsulfonyl)-cyclohexane in the form of white crystals (Melting Point: 113 to 115°C) (Yield: 84%).
(3) 2.93 ml of n-butyl lithium solution was added dropwise to 5 ml of THF solution having 930 mg of 4,4-dimethyl-1,1-(ethylenedioxy)-3-(phenylsulfonyl)-cyclohexane and 4 mg of triphenylmethane at -78°C under an argon stream. After stirring for 10 minutes, the reaction was effected at room temperature for one hour. 1 ml of HMPT was added thereto. The resulting solution was recooled to -78°C, and 2 ml of THF solution having 236 mg of 10-bromo-1-decanol was added dropwise. After reaction at -20°C for two hours, the reaction solution was poured into a saturated ammonium chloride solution, and extracted with ether. The organic phase was washed with water and saturated saline, and then dried over magnesium sulfate. The solvent was distilled off under reduced pressure. Then, the residue was purified by silica gel column chromatography using hexane-ethyl acetate, to thereby obtain 4,4-dimethyl-1,1-(ethylenedioxy)-3-(10-hydroxydecyl)-3-(phenylsulfonyl)-cyclohexane in the form of a colorless oil (TLC:(hexane-AcOEt:6-4)Rf=0.15) (Yield: 94%).
(4) 20 mg of paratoluenesulfonic acid was added to a solution having 400 mg of 4,4-dimethyl-1,1-(ethylenedioxy)-3-(10-hydroxydecyl)-3-(phenylsulfonyl)-cyclohexane in 30 ml of chloroform and 6 ml of acetone. The mixed solution was reacted at 50°C for 24 hours. 10 ml of saturated aqueous sodium bicarbonate solution was added thereto, and extracted with dichloromethane. The organic phase was washed with saturated saline, and then dried over magnesium sulfate. The solvent was distilled off under reduced pressure. Then, the residue was purified by silica gel column chromatography using hexane-ethyl acetate, to thereby obtain 4,4-dimethyl-3-(10-hydroxydecyl)-2-cyclohexen-1-one (4,4-dimethyl-3-(10-hydroxydecyl)-2-cyclohexenone) in the form of a colorless oil (TLC:(hexane-AcOEt:6-4)Rf=0.25) (Yield: 78%).

In a similar manner to Preparation Example 11, the following compounds were obtained.

### Preparation Example 12:

3-(11-hydroxyundecyl)-4,4-dimethyl-2-cyclohexen-1-one (3-(11-hydroxyundecyl)-4,4-dimethyl-2-cyclohexenone) (TLC:(hexane-AcOEt:6-4)Rf=0.25)

### Preparation Example 13:

3-(12-hydroxydodecyl)-4,4-dimethyl-2-cyclohexen-1-one (3-(12-hydroxydodecyl)-4,4-dimethyl-2-cyclohexenone) (TLC:(hexane-AcOEt:6-4)Rf=0.27)

### Preparation Example 14:

3-(13-hydroxytridecyl)-4,4-dimethyl-2-cyclohexen-1-one (3-(13-hydroxytridecyl)-4,4-dimethyl-2-cyclohexenone) (TLC:(hexane-AcOEt:6-4)Rf=0.3)

### Preparation Example 15:

3-(14-hydroxytetradecyl)-4,4-dimethyl-2-cyclohexen-1-one (3-(14-hydroxytetradecyl)-4,4-dimethyl-2-cyclohexenone) (TLC:(hexane-AcOEt:6-4)Rf=0.3)

### Preparation Example 16:

(1) 2.9 g of benzenesulfinic acid sodium was added to a solution having 1.5 g of 2-methyl-2-cyclohexen-1-one and 8 ml of water. 16 ml of 1N hydrochloric acid was added dropwise to this solution. After stirring at room temperature for 24 hours, the crystals thus deposited were filtered, and washed with water, isopropanol, and cold ether. After recrystallization with isopropanol, 2-methyl-3-(phenylsulfonyl)-cyclohexan-1-one was obtained in the form of white crystals (TLC:(hexane-AcOEt:6-4)Rf=0.25) (Yield: 93%).
(2) 0.41 ml of 1,2-ethanediol and 0.1 g of anhydrous paratoluenesulfonic acid were added to a solution having 1.4 g of 2-methyl-3-(phenylsulfonyl)-cyclohexan-1-one dissolved in 20 ml of benzene. The reaction solution was heated under reflux for four hours. After the reaction, 2M aqueous sodium bicarbonate solution was added thereto, and subjected to ethyl acetate extraction three times. The organic phase was washed with saturated saline, and then dried over magnesium sulfate. The solvent was distilled off under reduced pressure. Then, the residue was recrystallized with ether, to thereby obtain 1,1-(ethylenedioxy)-2-methyl-3-(phenylsulfonyl)-cyclohexane in the form of white crystals (Melting Point: 76 to 77°C) (Yield: 95%).
(3) 1.02 ml of n-butyl lithium solution was added dropwise to 5 ml of THF solution having 304 mg of 1,1-(ethylenedioxy)-2-methyl-3-(phenylsulfonyl)-cyclohexane and 4 mg of triphenylmethane at -78°C under an argon stream. After stirring for 10 minutes, the reaction was effected at room temperature for one hour. 1 ml of HMPT was added thereto. The resulting solution was recooled to -78°C, and 2 ml of THF solution having 90 mg of 10-bromo-1-decanol was added dropwise. After the reaction at -20°C for two hours, the reaction solution was poured into a saturated ammonium chloride solution. The solution was extracted with ether. The organic phase was washed with water and saturated saline, and then dried over magnesium sulfate. The solvent was distilled off under reduced pressure. Then, the residue was purified by silica gel column chromatography using hexane-ethyl acetate, to thereby obtain 1,1-(ethylenedioxy)-3-(10-hydroxydecyl)-2-methyl-3-(phenylsulfonyl)-cyclohexane in the form of a colorless oil (TLC:(hexane-AcOEt:6-4)Rf=0.2) (Yield: 92%).
(4) 20 mg of paratoluenesulfonic acid was added to a solution having 388 mg of 1,1-(ethylenedioxy)-3-(10-hydroxydecyl)-2-methyl-3-(phenylsulfonyl)-cyclohexane in 30 ml of chloroform and 6 ml of acetone. The mixed solution was effected a reaction at 50°C for 24 hours. 10 ml of saturated aqueous sodium bicarbonate solution was added thereto, and subjected to a dichloromethane extraction. The organic phase was washed with saturated saline, and then dried over magnesium sulfate. The solvent was distilled off under reduced pressure. Then, the residue was purified by silica gel column chromatography using hexane-ethyl acetate, to thereby obtain 3-(10-hydroxydecyl)-2-methyl-2-cyclohexen-1-one (3-(10-hydroxydecyl)-2-methyl-2-cyclohexenone) in the form of a colorless oil (TLC:(hexane-AcOEt:6-4)Rf=0.2) (Yield: 45%).

In a similar manner to Preparation Example 16, the following compounds were obtained.

### Preparation Example 17:

3-(11-hydroxyundecyl)-2-methyl-2-cyclohexen-1-one (3-(11-hydroxyundecyl)-2-methyl-2-cyclohexenone) (TLC:(hexane-AcOEt:6-4)Rf=0.24)

### Preparation Example 18:

3-(12-hydroxydodecyl)-2-methyl-2-cyclohexen-1-one (3-(12-hydroxydodecyl)-2-methyl-2-cyclohexenone) (TLC:(hexane-AcOEt:6-4)Rf=0.26)

### Preparation Example 19:

3-(13-hydroxytridecyl)-2-methyl-2-cyclohexen-1-one (3-(13-hydroxytridecyl)-2-methyl-2-cyclohexenone) (TLC:(hexane-AcOEt:6-4)Rf=0.28)

### Preparation Example 20:

3-(14-hydroxytetradecyl)-2-methyl-2-cyclohexen-1-one (3-(14-hydroxytetradecyl)-2-methyl-2-cyclohexenone) (TLC:(hexane-AcOEt:6-4)Rf=0.3)

### Preparation Example 21:

(1) 4 ml of hexane solution withw-butyl lithium (1.4 M) was added to dry THF solution (8 ml) containing 1 g of 1-phenylsulfonylmethyl-2,6,6-trimethyl-1-cyclohexene and 4 mg of triphenylmethane at -78°C under an argon gas atmosphere. After stirring for 10 minutes, 1.5 ml of hexamethylphosphoric triamide was added under stirring at room temperature. After 1.5 hours at this temperature, the mixture was cooled to -78°C and 439 mg of 11-bromoundecanol was added slowly. The mixture was stirred for three hours at -20°C and poured into 40 ml of saturated ammonium chloride solution. The obtained solution was extracted with ether. The organic phase was washed with saline, and then dried over magnesium sulfate. The solvent was distilled off under reduced pressure. The residue was purified by silica gel column chromatography, to thereby obtain 622 mg of 1-(12-hydroxydodecyl-1-phenylsulfonyl)-2,6,6-trimethyl-1-cyclohexene as a white solid (TLC:(hexane-AcOEt:6-4)Rf=0.43).
(2) 366 mg of Na₂HPO₄ and 4 g of mercury-sodium amalgam were added to 25 ml of dry ethanol solution containing 579 mg of 1-(12-hydroxydodecyl-1-phenylsulfonyl)-2,6,6-trimethyl-1-cyclohexene at 0°C under an argon gas atmosphere. The mixture was stirred at room temperature for one hour, then cooled with 5% HCl, and extracted with ether. The organic phase was washed with water, and then dried over magnesium sulfate. The solvent was distilled off under reduced pressure. The hydroxyl group of the residue was acetylated according to the usual method, to thereby obtain 353 mg of 1-(12-acetoxydodecyl)-2,6,6-trimethyl-1-cyclohexene as a colorless oil (TLC:(hexane-AeOEt:5-5)Rf=0.75).
(3) 0.8 ml of water, 1.3 mg of ruthenium trichloride hydrate, and 1.26 ml of 70% t-BuOOH was added to 6 ml of cyclohexane solution containing 321 mg of 1-(12-acetoxydodecyl)-2,6,6-trimethyl-1-cyclohexene. The solution was stirred at room temperature for six hours, and was filtered through celite. The filtrate was added to a 10% Na₂SO₃ solution. The solution was extracted with ether, and the organic phase was washed with saline, and dried over magnesium sulfate. Then, the solvent was distilled off under reduced pressure. The residue was purified by silica gel column chromatography, to thereby obtain 227 mg of 3-(12-acetoxydodecyl)-2,4,4-trimethyl-2-cyclohexen-1-one as a colorless oil (TLC:(hexane-AcOEt:3-7)Rf=0.68).
(4) To dry methanol solution (8 ml) containing 132 mg of 3-(12-acetoxydodecyl)-2,4,4-trimethyl-2-cyclohexen-1-one was added 3 drops of water and 74 mg of K₂CO₃. After stirring at room temperature for 2.5 hours, pH of the solution was adjusted to pH 7 with 5% HCl. The mixture was extracted with ether, and the organic phase was dried over magnesium sulfate. The solvent was distilled off under reduced pressure. The residue was purified by silica gel column chromatography, to thereby obtain 94 mg of 3-(12-hydroxydodecyl)-2,4,4-trimethyl-2-cyclohexen-1-one (3-(12-hydroxydodecyl)-2,4,4,-trimethyl-2-cyclohexenone) as a colorless oil (TLC:(hexane-AcOEt:7-3)Rf=0.2).

In a similar manner to Preparation Example 21, the following compounds were obtained.

### Preparation Example 22:

3-(13-hydroxytridecyl)-2,4,4-trimethyl-2-cyclohexen-1-one (3-(13-hydroxytridecyl)-2,4,4-trimethyl-2-cyclohexenone) (TLC:(hexane-AcOEt:7-3)Rf--0.2)

### Preparation Example 23:

3-(14-hydroxytetradecyl)-2,4,4-trimethyl-2-cyclohexen-1-one (3-(14-hydroxytetradecyl)-2,4,4-trimethyl-2-cyclohexenone) (TLC:(hexane-AcOEt:7-3)Rf=0.25)

### Preparation Example 24:

3-(15-hydroxypentadecyl)-2,4,4-trimethyl-2-cyclohexen-1-one (3-(15-hydroxypentadecyl)-2,4,4-trimethyl-2-cyclohexenone) (TLC:(hexane-AcOEt:7-3)Rf=0.29)

### Preparation Example 25:

3-(16-hydroxyhexadecyl)-2,4,4-trimethyl-2-cyclohexen-1-one (3-(16-hydroxyhexadecyl)-2,4,4-trimethyl-2-cyclohexenone) (TLC:(hexane-AcOEt:7-3)Rf=0.26).

### [Example 1] (Analgesic effect in partial nerve injury models)

### [Test Method]

For a hyperalgesia model animal, sciatic nerve entrapment model rats (Bennett model rats) serving as partial nerve injury models, were used. That is, four-week-old male Spraugue-Dawley rats (Japan SLC, Inc.) were housed under a constant temperature and humidity condition for a week. Then, under anesthesia with pentobarbital (50 mg/kg), the sciatic nerve in the left hind paw was stripped from tissue, and raised using tweezers to slightly stretch the axon part. Next, as shown in Fig. 1, equally spaced four ligatures were loosely tied around the stripped sciatic nerve axon of about 1 cm, using an absorbable synthetic suture, to form a nerve injured paw. The right hind paw was left untreated for use as a normal paw. The operated site was disinfected with Isodine everyday in the first post-operative week to prevent infection.

As the test substance, 3-(15-hydroxypentadecyl)-2,4,4-trimethyl-2-cyclohexen-1-one (hereafter, also referred to as compound 24) synthesized in Preparation Example 24 was used.

Rats showing reduction of pain threshold by 30% or more in the nerve-injured paw, and change in pain threshold within a range of 5% in the normal paw, as compared to preoperative values, were used for the experiment. Prior to administration of the test substance, the nerve-injured paw (operated side) and the normal paw (untreated side) of each individual were subjected to an analgesic test. Furthermore, the test substance-administered group was intraperitoneally administered with a single dose of the test substance (8 mg/kg, 24 mg/kg, and 40 mg/kg) everyday for a week. The control group was administered with the vehicle of the test substance during the same period. After one week from the start of administration, the nerve-injured paw (operated side) and the normal paw (untreated side) of each rat were again subjected to an analgesic test.

The paw pressure (Randall-Selitto) method using pressure stimuli was employed as the analgesic test. Mechanical pressure stimuli were applied on the dorsal portion of the rat hind paw by an analgesimeter, and the minimum value of pressure stimulus at which vocalization or withdrawal occurred was defined as pain threshold. In the measurement of pain threshold according to the paw pressure method, a rat has to be held down and restrained. Therefore, the rats were handled for a week prior to the test so as to make them get used to the restraint, and care was taken to maintain the natural posture during the measurement.

### [Results and Discussion]

The group administered with the test substance (24 mg/kg and 40 mg/kg) once a day for a week, showed significant increase in pain threshold of the nerve-injured paw. The control group showed no significant change in pain threshold (Fig. 2). Moreover, the reduction of pain threshold shown in the nerve-injured paw by the sciatic nerve ligation operation was not observed in the normal paw. Furthermore, no significant change in pain threshold was shown in both of the test substance-administered group and the control group, even after the administration period.

Since hyperalgesia in a Bennett model is caused by loss of myelin, the test substance is considered to have a repair-promoting effect on myelin loss. Moreover, the test substance did not affect the pain threshold of the normal hind paw, and thus was proven to have an effect on the injured sensory nerve only and no effect on the normal nerve.

### [Example 2] (Injury-preventive effect in partial nerve injury models)

### [Test Method]

For a hyperalgesia model animal, sciatic nerve entrapment model rats (Bennett model rats) serving as partial nerve injury models, were used. That is, four-week-old male Spraugue-Dawley rats (Japan SLC, Inc.) were housed under a constant temperature and humidity condition for a week. Then, under anesthesia with pentobarbital (50 mg/kg), the sciatic nerve in the left hind paw was stripped from tissue, and raised using tweezers to slightly stretch the axon part. Next, as shown in Fig. 1, equally spaced four ligatures were loosely tied around the stripped sciatic nerve axon of about 1 cm, using an absorbable synthetic suture, to form a nerve-injured paw. The right hind paw was left untreated and used as a normal paw. The operated site was disinfected with Isodine everyday in the first post-operative week to prevent infection.

As the test substance, compound 24 synthesized in Preparation Example 24 was used.

The nerve-injured paw (operated side) and the normal paw (untreated side) of each individual were previously subjected to an analgesic test, subsequently followed by Bennett operation. Furthermore, a single dose of the test substance (8 mg/kg) was intraperitoneally administered from the day of the operation, everyday for a week. The control group was administered with the vehicle of the test substance during the same period. After one week from the start of administration, the nerve injured paw (operated side) and the normal paw (untreated side) of each rat were again subjected to an analgesic test.

The paw pressure (Randall-Selitto) method using pressure stimuli was employed as the analgesic test. Mechanical pressure stimuli were applied on the dorsal portion of the rat hind paw by an analgesimeter, and the minimum value of pressure stimulus at which vocalization or withdrawal occurred was defined as the pain threshold. In the measurement of pain threshold according to the Paw pressure method, a rat has to be held down and restrained. Therefore, the rats were handled for a week prior to the test so as to make them get used to the restraint, and care was taken to keep the natural posture during the measurement.

### [Results and Discussion]

The control group showed a significant reduction of pain threshold due to the Bennett operation. The group administered with the test substance (8 mg/kg) showed no significant change in pain threshold even after the Bennett operation (Fig. 3). Moreover, the reduction of pain threshold shown in the nerve-injured paw by the sciatic nerve ligation operation was not observed in the normal paw. Furthermore, no significant change in pain threshold was shown in both of the test substance-administration group and the control group, even after the administration period.

Since hyperalgesia in a Bennett model is caused by loss of myelin, the test substance is considered to have a suppressing effect on myelin loss. Moreover, the test substance did not affect the pain threshold of the normal hind paw, and thus was proven to have an effect on the injured sensory nerve only, with no effect on the normal nerve.

### [Example 3] (Improving effect in dullness models)

### [Test Method]

Four-week-old male ddY mice were administered with a single dose of streptozotocin (hereunder, also referred to as STZ) (200 mg/kg) by tail vein injection, everyday for a week. For 24 hours after STZ administration, the rats were allowed to freely take a 10% glucose solution instead of drinking water, and then allowed to freely take distilled water. As the test substance, compound 24 synthesized in Preparation Example 24 was used. Concurrently with the start of STZ administration, the test substance-administration group was intraperitoneally administered with a single dose of the test substance (8 mg/kg, 24 mg/kg, and 40 mg/kg) everyday for a week. The control group was administered with the vehicle of the test substance during the same period. The blood glucose level of the tail venous blood was measured by a blood glucose meter (DEXTER ZII: Bayer Medical Ltd.). Individuals with a blood sugar level of 400 mg/dL or more, were used for the experiment. After a week of STZ administration, the mice were subcutaneously injected with 1% formaldehyde solution (20 µL) into the plantar of the left hind paw. The total time spent for licking (licking of plantar of the left hind paw) behavior and biting (biting of plantar of the left hind paw) behavior was scored every 5 minutes after administration, for 60 minutes.

### [Results and Discussion]

Mice subcutaneously injected with 1% formaldehyde solution to their plantar showed a biphasic pain response having peaks at 0-5 minutes and 15-25 minutes post-administration. At the first week of STZ administration, Phase II pain response was suppressed, showing hypesthesia. A week of intraperitoneal administration of the test substance with the STZ administration improved Phase II torpor (Fig. 4). Since the test substance has an effect of extending neurites, the hypesthesia may have been improved due nerve fiber repair.

### Industrial Applicability

The present invention provides either preventive or therapeutic agents for a sensory disorder wherein the sensory disorder is reduction of pain threshold caused by nerve injury, or both, which agents comprise, as an active ingredient, a cyclohexenone long chain alcohol. Accordingly, the present invention can be applied to medicines and foods for preventing or treating a sensory disorder wherein the sensory disorder is reduction of pain threshold caused by nerve injury.

## Claims

1. A compound for use in treating and/or preventing a sensory disorder, wherein said compound is a cyclohexenone long chain alcohol compound represented by the following formula (1): 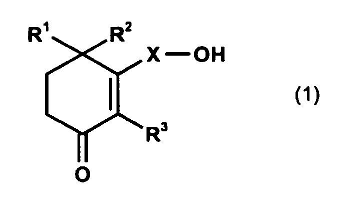 wherein
R¹, R², and R³ each represent a hydrogen atom or a methyl group, and
X represents a linear or branched C₁₀-C₂₈ alkylene or alkenylene'group; and
wherein the sensory disorder is reduction of pain threshold caused by nerve injury.

2. Use of a compound for the preparation of a medicament for treating and/or preventing a sensory disorder, wherein said compound is a cyclohexenone long chain alcohol compound represented by the following formula (1): 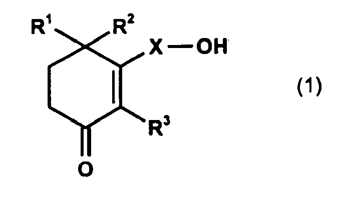 wherein
R¹, R², and R³ each represent a hydrogen atom or a methyl group, and
X represents a linear or branched C₁₀-C₂₈ alkylene or alkenylene group; and
wherein the sensory disorder is reduction of pain threshold caused by nerve injury.

3. The compound for use according to claim 1 or use according to claim 2, wherein R¹ is a methyl group and X is a linear C₁₀-C₂₈ alkylene group.

4. The compound for use or use according to claim 3, wherein R² is a methyl group.

5. The compound for use or use according to claim 3 or 4, wherein R³ is a methyl group.

6. The compound for use according to claim 1 or use according to claim 2, wherein R¹ and R² are hydrogen atoms.

7. The compound for use according to claim 1 or use according to claim 2, wherein the cyclohexenone long chain alcohol compound is selected from the group consisting of the following compounds:
3-(10-hydroxydecyl)-2-cyclohexen-1-one;
3-(11-hydroxyundecyl)-2-cyclohexen-1-one;
3-(12-hydroxydodecyl)-2-cyclohexen-1-one;
3-(13-hydroxytridecyl)-2-cyclohexen-1-one;
3-(14-hydroxytetradecyl)-2-cyclohexen-1-one;
3-(10-hydroxydecyl)-4-methyl-2-cyclohexen-1-one;
3-(11-hydroxyundecyl)-4-methyl-2-cyclohexen-1-one;
3-(12-hydroxydodecyl)-4-methyl-2-cyclohexen-1-one;
3-(13-hydroxytridecyl)-4-methyl-2-cyclohexen-1-one;
3-(14-hydroxytetradecyl)-4-methyl-2-cyclohexen-1-one;
4,4-dimethyl-3-(10-hydroxydecyl)-2-cyclohexen-1-one;
3-(11-hydroxyundecyl)-4,4-dimethyl-2-cyclohexen-1-one;
3-(12-hydroxydodecyl)-4,4-dimethyl-2-cyclohexen-1-one;
3-(13-hydroxytridecyl)-4,4-dimethyl-2-cyclohexen-1-one;
3-(14-hydroxytetradecyl)-4,4-dimethyl-2-cyclohexen-1-one;
3-(10-hydroxydecyl)-2-methyl-2-cyclohexen-1-one;
3-(11-hydroxyundecyl)-2-methyl-2-cyclohexen-1-one;
3-(12-hydroxydodecyl)-2-methyl-2-cyclohexen-1-one;
3-(13-hydroxytridecyl)-2-methyl-2-cyclohexen-1-one;
3-(14-hydroxytetradecyl)-2-methyl-2-cyclohexen-1-one;
3-(12-hydroxydodecyl)-2,4,4-trimethyl-2-cyclohexen-1-one 3-(13-hydroxytridecyl)-2,4,4-trimethyl-2-cyclohexen-1-one;
3-(14-hydroxytetradecyl)-2,4,4-trimethyl-2-cyclohexen-1-one;
3-(15-hydroxypentadecyl)-2,4,4-trimethyl-2-cyclohexen-1-one; and
3-(16-hydroxyhexadecyl)-2,4,4-trimethyl-2-cyclohexen-1-one.

## Patentansprüche

1. Verbindung zur Verwendung zum Behandeln und/oder Verhüten einer sensorischen Störung, wobei die Verbindung eine langkettige Cyclohexenon-Alkohol-Verbindung ist, die durch die folgende Formel (1) wiedergegeben ist: 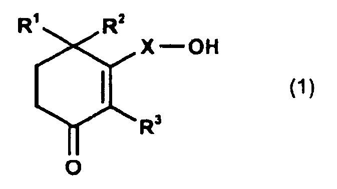 wobei
R¹, R² und R³ jeweils ein Wasserstoffatom oder eine Methylgruppe bedeuten, und
X eine lineare oder verzweigte C₁₀-C₂₈-Alkylen- oder Alkenylengruppe bedeutet; und
wobei es sich bei der sensorischen Störung um eine Verringerung der Schmerzschwelle handelt, die durch eine Nervenverletzung verursacht ist.

2. Verwendung einer Verbindung für die Herstellung eines Medikaments für das Behandeln und/oder Verhüten einer sensorischen Störung, wobei die Verbindung eine langkettige Cyclohexenon-Alkohol-Verbindung ist, die durch die folgende Formel (1) wiedergegeben ist: 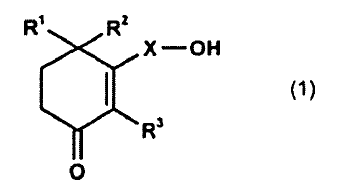 wobei
R¹, R² und R³ jeweils ein Wasserstoffatom oder eine Methylgruppe bedeuten, und
X eine lineare oder verzweigte C₁₀-C₂₈-Alkylen- oder Alkenylengruppe bedeutet; und wobei es sich bei der sensorischen Störung um eine Verringerung der Schmerzschwelle handelt, die durch eine Nervenverletzung verursacht ist.

3. Verbindung zur Verwendung nach Anspruch 1 oder Verwendung nach Anspruch 2, wobei R¹ eine Methylgruppe ist und X eine lineare C₁₀-C₂₈-Alkylengruppe ist.

4. Verbindung zur Verwendung oder Verwendung nach Anspruch 3, wobei R² eine Methylgruppe ist.

5. Verbindung zur Verwendung oder Verwendung nach Anspruch 3 oder 4, wobei R³ eine Methylgruppe ist.

6. Verbindung zur Verwendung nach Anspruch 1 oder Verwendung nach Anspruch 2, wobei R¹ und R² Wasserstoffatome sind.

7. Verbindung zur Verwendung nach Anspruch 1 oder Verwendung nach Anspruch 2, wobei die langkettige Cyclohexenon-Alkohol-Verbindung ausgewählt ist aus der Gruppe bestehend aus den folgenden Verbindungen:
3-(10-Hydroxydecyl)-2-cyclohexen-1-on;
3-(11-Hydroxyundecyl)-2-cyclohexen-1-on;
3-(12-Hydroxydodecyl)-2-cyclohexen-1-on;
3-(13-Hydroxytridecyl)-2-cyclohexen-1-on;
3-(14-Hydroxytetradecyl)-2-cyclohexen-1-on;
3-(10-Hydroxydecyl)-4-methyl-2-cyclohexen-1-on;
3-(11-Hydroxyundecyl)-4-methyl-2-cyclohexen-1-on;
3-(12-Hydroxydodecyl)-4-methyl-2-cyclohexen-1-on;
3-(13-Hydroxytridecyl)-4-methyl-2-cyclohexen-1-on;
3-(14-Hydroxytetradecyl)-4-methyl-2-cyclohexen-1-on;
4,4-Dimethyl-3-(10-hydroxydecyl)-2-cydohexen-1-on;
3-(11-Hydroxyundecyl)-4,4-dimethyl-2-cyclohexen-1-on;
3-(12-Hydroxydodecyl)-4,4-dimethyl-2-cyclohexen-1-on;
3-(13-Hydroxytridecyl)-4,4-dimethyl-2-cyclohexen-1-on;
3-(14-Hydroxytetradecyl)-4,4-dimethyl-2-cyclohexen-1-on;
3-(10-Hydroxydecyl)-2-methyl-2-cyclohexen-1-on;
3-(11-Hydroxyundecyl)-2-methyl-2-cyclohexen-1-on;
3-(12-Hydroxydodecyl)-2-methyl-2-cyclohexen-1-on;
3-(13-Hydroxytridecyl)-2-methyl-2-cyclohexen-1-on;
3-(14-Hydroxytetradecyl)-2-methyl-2-cyclohexen-1-on;
3-(12-Hydroxydodecyl)-2,4,4-trimethyl-2-cyclohexen-1-on;
3-(13-Hydroxytridecyl)-2,4,4-trimethyl-2-cyclohexen-1-on;
3-(14-Hydroxytetradecyl)-2,4,4-trimethyl-2-cyclohexen-1-on;
3-(15-Hydroxypentadecyl)-2,4,4-trimethyl-2-cyclohexen-1-on; und
3-(16-Hydroxyhexadecyl)-2,4,4-trimethyl-2-cyclohexen-1-on.

## Revendications

1. Composé pour une utilisation dans le traitement et/ou la prévention d'un trouble sensoriel, dans laquelle le composé est un composé alcoolique à chaîne longue de cyclohexénone représenté par la formule (1) suivante : 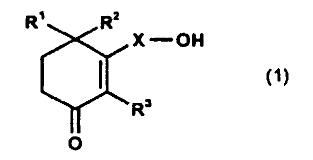 dans laquelle
R¹, R² et R³ représentent chacun un atome d'hydrogène ou un groupe méthyle, et
X représente un groupe alkylène ou alcénylène en C₁₀-C₂₈ linéaire ou ramifié ; et
dans laquelle le trouble sensoriel est une réduction du seuil de douleur provoquée par une lésion nerveuse.

2. Utilisation d'un composé pour la préparation d'un médicament pour traiter et/ou prévenir un trouble sensoriel, dans laquelle le composé est un composé alcoolique à chaîne longue de cyclohexénone représenté par la formule (1) suivante : 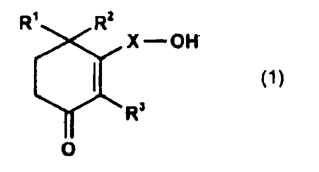 dans laquelle
R¹, R² et R³ représentent chacun un atome d'hydrogène ou un groupe méthyle, et
X représente un groupe alkylène ou alcénylène en C₁₀-C₂₈ linéaire ou ramifié ; et
dans laquelle le trouble sensoriel est une réduction du seuil de douleur provoquée par une lésion nerveuse.

3. Composé pour une utilisation selon la revendication 1 ou utilisation selon la revendication 2, dans lesquels R¹ est un groupe méthyle et X est un groupe alkylène linéaire en C₁₀-C₂₈.

4. Composé pour une utilisation ou utilisation selon la revendication 3, dans lesquels R² est un groupe méthyle.

5. Composé pour une utilisation ou utilisation selon la revendication 3 ou 4, dans lesquels R³ est un groupe méthyle.

6. Composé pour une utilisation selon la revendication 1 ou utilisation selon la revendication 2, dans lesquels R¹ et R² sont des atomes d'hydrogène.

7. Composé pour une utilisation selon la revendication 1 ou utilisation selon la revendication 2, dans lesquels le composé alcoolique à chaîne longue de cyclohexénone est choisi dans le groupe constitué par les composés suivants :
3-(10-hydroxydécyl)-2-cyclohexén-1-one;
3-(11-hydroxyundécyl)-2-cyclohexén-1-one;
3-(12-hydroxydodécyl)-2-cyclohexén-1-one;
3-(13-hydroxytridécyl)-2-cyclohexén-1-one;
3-(14-hydroxytétradécyl)-2-cyclohexén-1-one;
3-(10-hydroxydécyl)-4-méthyl-2-cyclohexén-1-one;
3-(11-hydroxyundécyl)-4-méthyl-2-cyclohexén-1-one;
3-(12-hydroxydodécyl)-4-méthyl-2-cyclohexén-1-one;
3-(13-hydroxytridécyl)-4-méthyl-2-cyclohexén-1-one;
3-(14-hydroxytétradécyl)-4-méthyl-2-cyclohexén-1-one;
4,4-diméthyl-3-(10-hydroxydécyl)-2-cyclohexén-1-one;
3-(11-hydroxyundécyl)-4,4-diméthyl-2-cyclohexén-1-one;
3-(12-hydroxydodécyl)-4,4-diméthyl-2-cyclohexen-1-one:
3-(13-hydroxytridécyl)-4,4-diméthyl-2-cyclohexén-1-one;
3-(14-hydroxytétradécyl)-4,4-diméthyl-2-cyclohexén-1-one;
3-(10-hydroxydécyl)-2-méthyl-2-cyclohexén-1-one;
3-(11-hydroxyundécyl)-2-méthyl-2-cyclohexén-1-one;
3-(12-hydroxydodécyl)-2-méthyl-2-cyclohexén-1-one;
3-(13-hydroxytridécyl)-2-méthyl-2-cyclohexén-1-one;
3-(14-hydroxytétradécyl)-2-méthyl-2-cyclohexén-1-one;
3-(12-hydroxydodécyl)-2,4,4-triméthyl-2-cyclohexén-1-one;
3-(13-hydroxytridécyl)-2,4,4-triméthyl-2-cyclohexén-1-one;
3-(14-hydroxytétradécyl)-2,4,4-triméthyl-2-cyclohexén-1-one;
3-(15-hydroxypentadécyl)-2,4,4-triméthyl-2-cyclohexén-1-one; et
3-(16-hydroxyhexadécyl)-2,4,4-triméthyl-2-cyclohexén-1-one.
